# EUROPÄISCHE PATENTSCHRIFT

(11) **EP 2 195 411 B1**
(45) Veröffentlichungstag und Bekanntmachung des Hinweises auf die Patenterteilung: **31.07.2019**
(21) Anmeldenummer: 08804553.9
(22) Anmeldetag: 22.09.2008
(51) Int. Cl.: C12M 1/107, B01F 13/00

(54) **VERFAHREN ZUR HERSTELLUNG VON BIOGAS**
METHOD FOR PRODUCING BIOGAS
PROCÉDÉ POUR FABRIQUER DU BIOGAZ

(30) Priorität: 25.09.2007 CH 14852007
(43) Veröffentlichungstag der Anmeldung: 16.06.2010
(73) Patentinhaber: Inauen, Urs, 9050 Appenzell (CH)
(72) Erfinder: Inauen, Urs, 9050 Appenzell (CH)
(74) Vertreter: Liebetanz, Michael
(86) Internationale Anmeldenummer: PCT/EP2008/062627
(87) Internationale Veröffentlichungsnummer: WO 2009/040330

(56) Entgegenhaltungen:
- WO-A-2007/077577
- DE-A1- 3 012 198
- JP-A- 11 188 255
- US-A- 3 954 619
- US-A- 4 090 940
- US-A- 4 880 547

## Beschreibung

### Technisches Gebiet der Erfindung

Die vorliegende Erfindung betrifft ein Verfahren zur Herstellung von Biogas gemäss den Merkmalen des Oberbegriffs des Anspruchs 1 sowie zur Verwendung in einem solchen Verfahren besonders ausgestaltete Mischkörper.

### Stand der Technik

Aus dem Stand der Technik sind Biogasreaktoren, Vergärbottiche oder Gärbehälter bekannt. Die Reaktoren werden mit vergärbarer Biomasse, wie Reststoffe (Klärschlamm, Bioabfall, Speisereste), Wirtschaftsdünger (Gülle, Mist) oder mit anderen geeigneten Materialien befüllt. Die vergärbare Biomasse oder die vergärbaren Materialien können einen flüssigen und/oder einen festen Zustand aufweisen. Durch die Vergärung der genannten Materialien kann ein Biogas gewonnen werden, welches beispielsweise zum Antrieb von Gas-Generatoren, Kraftfahrzeugen oder zur Verbrennung in einer Heizung eingesetzt werden kann.

Ein solcher Reaktor hat üblicherweise die Form eines zylindrischen Behälters, welcher mit einem Deckel verschlossen ist. Der Reaktor wird mit Biomasse befüllt, aus welchem dann das Biogas gewonnen werden kann. Dabei strömt das Biogas aus der Biomasse nach oben, wo es im Bereich des Deckels dem Reaktor entnommen werden kann. Ferner umfasst ein solcher Reaktor ein Rührwerk, welches die vergärbaren Materialien konstant umrührt. Zudem können Heizelemente vorgesehen werden, welche die Erhitzung der Biomasse die Vergärung zusätzlich beschleunigen bzw. unterstützen.

Üblicherweise befinden sich die flüssigen Bestandteile der vergärbaren Materialien im Bereich des Bodens, und auf diesen flüssigen Bestandteilen schwimmen feste Bestandteile mit geringerer Dichte auf. Diese schwimmende Schicht ist nachteilig für den Wirkungsgrad der Vergärung bzw. Gasbildung. Man hat deshalb bei Vorrichtungen des Standes der Technik Flügelrührwerke vorgesehen, welche diese Schwimmschicht teilweise durchbrechen. Jedoch ist die Wirkung von einer solchen teilweisen Durchbrechung häufig unbefriedigend, da sich die Schwimmschicht immer wieder von neuem bildet.

Aus der WO 2007/077577 ist ein Kugelball bekannt geworden, welcher Bakterien umfasst, die an Wasser zwecks Reinigung desselben abgegeben werden.

### Darstellung der Erfindung

Ausgehend von diesem Stand der Technik liegt der Erfindung eine Aufgabe zugrunde, ein Verfahren anzugeben, welches den Wirkungsgrad von Biogasreaktoren verbessert. Ferner soll ein Verfahren angegeben werden, welches in einfacher Art und Weise bei schon bestehenden Biogasreaktoren eingesetzt werden kann.

Diese Aufgabe löst Verfahren zur Herstellung von Biogas mit den Merkmalen des Patentanspruchs 1.

Eine weitere Aufgabe ist es, einen speziell für die Anwendung in einem solchen Verfahren ausgebildeten Mischkörper anzugeben. Diese Aufgabe löst ein Mischkörper mit den Merkmalen der Patentansprüche 4.

Demgemäss wird bei einem Verfahren zur Herstellung von Biogas, bei welchem Biomasse in einen Biogasreaktor eingefüllt wird, eine Vielzahl von Mischkörpern auf der Biomasse angeordnet. Die Mischkörper schwimmen auf der Oberfläche der Biomasse und tauchen dabei teilweise in die Biomasse ein.

Vorzugsweise ist des weiteren eine Mischvorrichtung vorhanden, die geeignet ist, die Biomasse aktiv zu durchmischen, z.B. ein herkömmliches Rührwerk, oder die Biomasse wird auf andere Weise in Bewegung versetzt. Durch die Anwesenheit der Mischkörper wird die Oberfläche der Biomasse im Biogasreaktor dann besonders effizient durchmischt, da die Hohlkörper aufgrund der sich in Bewegung befindlichen Biomasse ebenfalls in Bewegung versetzt werden. Insbesondere wird die Bildung einer Schwimmschicht auf der Biomasse verhindert. Dies beeinflusst den Wirkungsgrad der Gasgewinnung in einem Biogasreaktor positiv. Ferner wird die Oberfläche der Biomasse zusätzlich vergrössert, was den Wirkungsgrad ebenfalls positiv beeinflusst.

Vorzugsweise überdecken die Mischkörper zwischen 20% bis 85%, insbesondere 30% bis 70% der Oberfläche der Biomasse.

Bevorzugterweise sind die Mischkörper im wesentlichen als Hohlkörper ausgebildet.

Vorzugsweise weisen die Mischkörper eine Oberflächenstruktur auf. Dabei gestaltet sich der Durchmischungsvorgang noch effektiver, da die sich in Bewegung befindliche Biomasse an der Oberflächenstruktur besonders gut angreifen kann, somit wird für eine gute Durchmischung der Biomasse gesorgt.

Die Mischkörper sind im wesentlichen kugelförmig ausgebildet. Dies hat den Vorteil, dass die Mischkörper durch die Bewegung der Biomasse gut mitbewegt werden und sich leicht um beliebige Achsen drehen können.

Der Mischkörper ist als Hohlkörper mit mindestens einer Öffnung ausgebildet, wobei die mindestens eine Öffnung ein Eintreten bzw. ein Austreten von Biomasse in den Hohlkörper erlaubt. Alternativ kann der Hohlkörper auch ohne Öffnung ausgebildet sein, wobei dieser Hohlkörper auch als geschlossener Hohlkörper bezeichnet werden kann.

Die durch die Öffnung in den Hohlkörper eintretende Biomasse kann im Hohlkörper ebenfalls vergären. Zudem haften Teile der Biomasse bei einer Drehung des Hohlkörpers an der Wand an, was die Vergärung zusätzlich beschleunigt.

Vorzugsweise umfasst der Hohlkörper mindestens eine weitere Öffnung. Hierdurch kann innerhalb des gleichen Zeitraumes mehr Biomasse in den Hohlkörper eintreten, was den Wirkungsgrad weiterhin erhöht.

Bevorzugterweise ist die weitere Öffnung im wesentlichen diametral gegenüber der ersten Öffnung angeordnet, wodurch der Mischkörper gut ausbalanciert ist.

Vorzugsweise weist der Mischkörper mindestens ein Mischelement auf, welches auf der nach aussen zugewandten Oberfläche der Wandung angeordnet ist und von dieser absteht. Das Mischelement sorgt für eine bessere Durchmischung der Biomasse bzw. Durchtrennung einer eventuellen Schwimmschicht und dient gleichzeitig als Mitnehmerelement, so dass der Mischkörper gut von der sich bewegenden Biomasse antreiben lässt. Das Mischelement lässt sich sowohl am geschlossenen Mischkörper bzw. Hohlkörper als auch am Mischkörper bzw. Hohlkörper mit mindestens einer Öffnung anbringen.

Vorzugsweise umfasst der Mischkörper mindestens einen Auftriebskörper, welcher verhindert, dass die Mischvorrichtung vollständig absinken kann. Zudem orientiert sich der Mischkörper in der Biomasse vorzugsweise immer so, dass Teile von mindestens einer Öffnung in die Biomasse eintauchen. Dabei wird sichergestellt, dass die Biomasse in den Hohlkörper eindringen kann.

Vorzugsweise ist der mindestens eine Auftriebskörper im Inneren des Hohlkörpers angeordnet. Der mindestens eine Auftriebskörper kann sowohl beim geschlossenen Mischkörper bzw. Hohlkörper als auch beim Hohlkörper mit mindestens einer Öffnung vorhanden sein. Zusätzlich können auch bei einer Ausführung mit Auftriebskörper ein oder mehrere Mischelemente auf der Oberfläche des Mischkörpers angeordnet sein.

Der mindestens eine Auftriebskörper weist vorzugsweise einen Hohlraum auf, welcher mit einem Medium befüllt ist, das eine geringere Dichte als die Biomasse aufweist.

Vorzugsweise ist das Medium, mit welchem der mindestens eine Auftriebskörper befüllt ist, ein Gas, insbesondere Luft, oder der Auftriebskörper kann aus einem geschäumten Kunststoff gefertigt sein.

Vorzugsweise ist der mindestens eine Auftriebskörper einstückig mit der Wandung des Hohlkörpers ausgebildet, wobei der Hohlkörper dann besonders effizient und kostengünstig hergestellt werden kann.

Weitere vorteilhafte Ausführungsformen sind in den Unteransprüchen angegeben.

### Kurze Beschreibung der Zeichnung

Bevorzugte Ausführungsformen werden im folgenden anhand der Zeichnung beispielhaft näher beschrieben. Es zeigen:
- Fig. 1: eine schematische Draufsicht von oben in einen Biogasreaktor, welcher mit erfindungsgemässen Mischkörpern ausgestattet ist;
- Fig. 2: eine schematische Ansicht eines Ausschnittes eines Biogasreaktors von der Seite;
- Fig. 3a: eine schematische Draufsicht eines erfindungsgemässen Mischkörpers gemäss einem ersten Ausführungsbeispiel;
- Fig. 3b: eine Schnittdarstellung des Mischkörpers nach Fig. 3a;
- Fig. 3c: eine Vorderansicht des Mischkörpers nach Fig. 3a;
- Fig. 4a: eine schematische Draufsicht eines erfindungsgemässen Mischkörpers gemäss einem zweiten Ausführungsbeispiel;
- Fig. 4b: eine Schnittdarstellung des Mischkörpers nach Fig. 4a; und
- Fig. 4c: eine Vorderansicht des Mischkörpers nach Fig. 4a.

### Beschreibung von bevorzugten Ausführungsbeispielen

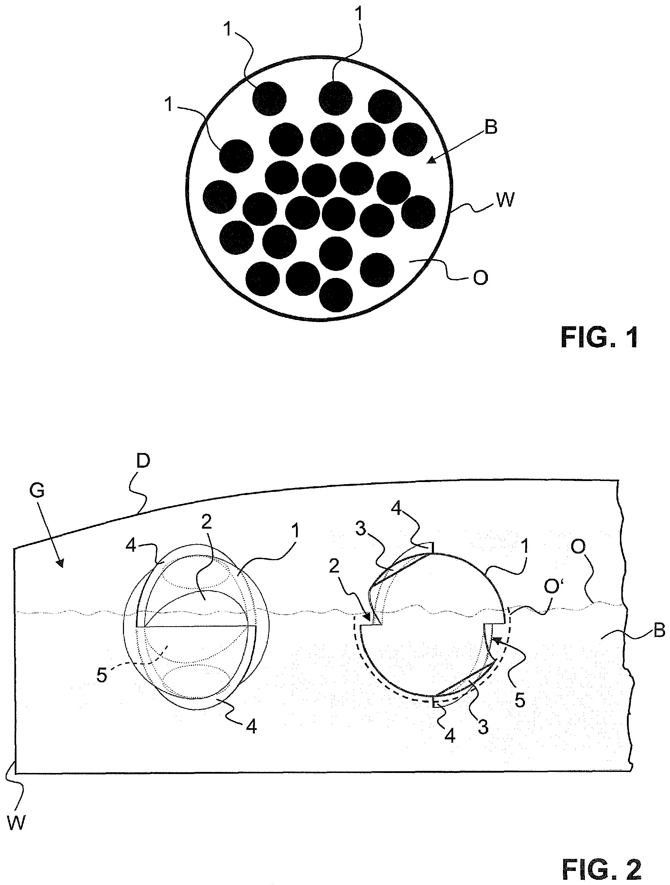

Die Figur 1 zeigt in einer schematischen Darstellung eine Draufsicht der Innenseite eines Biogasreaktors oder eines Vergärbottichs. Typischerweise verfügen solche Biogasreaktoren über Oberflächen von mehreren 100 m². Der Biogasreaktor ist hier nur schematisch dargestellt und ist im wesentlichen mit einer zylindrischen Seitenwand W begrenzt. Der Biogasreaktor ist mit Biomasse B befüllt. Eine Vielzahl Mischkörper 1 sind zur Verbesserung des Wirkungsgrades eines Biogasreaktors auf der Oberfläche O der Biomasse B angeordnet und tauchen teilweise in diese ein. Vorzugsweise werden derart viele Mischkörper 1 platziert, dass die Mischkörper 1 zwischen 20% bis 85%, insbesondere 30% bis 70% der Oberfläche O der Biomasse B überdecken.

Die Figur 2 zeigt einen Ausschnitt aus dem besagten Biogasreaktor in einer Seitenansicht mit zwei Mischkörpern 1. Hier ist schematisch angedeutet, dass der Biogasreaktor ferner einen Deckel D aufweist. Die Biomasse B befindet sich im wesentlichen im unteren Bereich des Biogasreaktors, während sich im oberen Bereich ein Gasgemisch G aus Biogas und/oder Luft befindet. Dieses Gasgemisch kann mittels einer nicht gezeigten Entnahmestelle aus dem Biogasreaktor entnommen werden. Der auf der rechten Seite dargestellte Mischkörper 1 ist als Schnittdarstellung abgebildet, während der auf der linken Seite dargestellte als Vorderansicht dargestellt ist. Der Mischkörper 1 ist im wesentlichen als Hohlkörper ausgebildet. Der Hohlkörper weist eine Wandung 10 auf, die einen Hohlraum 11 zur Aufnahme von Luft, Biogas und/oder Biomasse begrenzt, und kann verschiedenartig ausgebildet sein. Der Hohlkörper ist so ausgestaltet, dass er auf der Biomasse B aufschwimmt bzw. teilweise in diese eintaucht. Ferner kann der Hohlkörper mit mindestens einer Öffnung 2 ausgestattet sein.

Durch Bewegen eines Rührwerkes wird die gesamte Biomasse B in Bewegung versetzt. Aufgrund der resultierenden Strömung werden auch die Mischkörper 1 auf der Biomasse in Bewegung versetzt. Durch die Bewegung drehen sich die Mischkörper 1 um ihre eigene Achse, was zu einer Durchbrechung bzw. Durchmischung der Schwimmschicht auf der Biomasse B führt. Durch die Bewegung kann auch immer wieder Biomasse B bzw. Teile des Gasgemisches G in den Hohlkörper eintreten bzw. aus diesem wieder austreten. Dadurch wird die Durchmischung der Biomasse B beschleunigt. Ferner kann die Biomasse B auch in der Hohlkugel vergären.

Durch die Anwesenheit der sich frei bewegenden Mischkörper 1 erfolgt eine effiziente Durchmischung der gesamten Oberfläche der Biomasse B. Durch das Vorsehen der Mischkörper 1 wird das Entstehen einer Schwimmschicht auf der Oberfläche der Biomasse verhindert. Falls sich wider Erwarten dennoch eine Schwimmschicht bildet, wird diese entstehende Schwimmschicht stetig durch die Mischkörper 1 zerteilt. Dadurch kann der Vergärungsprozess positiv beeinflusst werden, was zu einer Steigerung des Wirkungsgrades der Gasgewinnung führt. Dies ist ein Vorteil gegenüber Vorrichtungen des Standes der Technik, insbesondere gegenüber reinen Flügelrührwerken, bei welchen die Schwimmschicht nur an lokalen Stellen durchbrochen wird.

Ferner bewirkt die Anwesenheit der Mischkörper 1 eine Vergrösserung der Oberfläche O der Biomasse B, wodurch die Vergärung der Biomasse B positiv beeinflusst bzw. beschleunigt wird. Die Vergrösserung der Oberfläche O wird dadurch bewirkt, dass der Hohlkörper 1 teilweise in die Biomasse B eintaucht bzw. hineinragt und bei einer Drehbewegung immer wieder Biomasse B auf seiner Oberfläche mitnimmt. Die Oberfläche der Biomasse B in Abwesenheit der Mischkörper 1 entspricht im wesentlichen der Projektion des Mischkörpers 1. Die Oberfläche O', welche durch die Anwesenheit des Mischkörpers geschaffen wird, entspricht der Oberfläche des Hohlkörpers, welche in die Biomasse eingetaucht ist. Durch das Anordnen wird also eine vergrösserte Oberfläche O' erzielt.

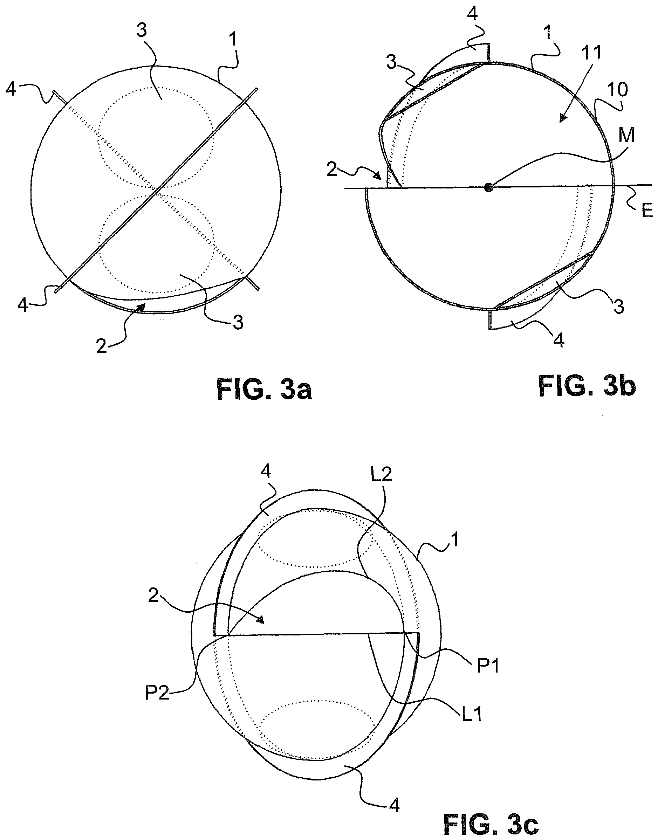

Die Figuren 3a bis 3c zeigen einen Mischkörper 1 gemäss einem ersten Ausführungsbeispiel. Der Mischkörper 1 weist hier im wesentlichen die Gestalt einer Hohlkugel auf. Durch das Vorhandensein der Öffnung 2 kann Biomasse durch die Öffnung 2 in den Hohlkörper 1 eintreten bzw. wieder austreten. Dies wird durch die Form der Öffnung 2 begünstigt, da durch die Drehung des Hohlkörpers 1 ein Art Schöpfbewegung entsteht. Da sich der Hohlkörper 1 kontinuierlich dreht, kann sich der Hohlraum 11 des Hohlkörpers 1 teilweise mit Biomasse füllen. Die sich im Hohlraum 1 befindliche Biomasse vergärt ebenfalls, was dazu führt, dass der Hohlraum 11 auch einen Teil des Gasgemisches G bzw. Biogas enthält. Bei weiteren Bewegungen des Hohlkörpers 1 wird dieser Teil Biogas bzw. die vergärende Biomasse wieder in die Biomasse B untergemischt, welche sich im Bioreaktor befindet. Dadurch wird die Vergärung beschleunigt.

Die Öffnung 2 erstreckt sich über einen definierten Oberflächenabschnitt und wird durch eine erste Linie L1 und eine zweite Linie L2 begrenzt. Die erste Linie L1 ist die Schnittlinie auf der Oberfläche des Hohlkörpers 1, hier von der Hohlkugel 1, mit einer Ebene E, welche durch den Mittelpunkt M der Hohlkugel 1 verläuft. Das heisst mit anderen Worten, dass die Linie L1 äquatorial verläuft. Dabei erstreckt sich die Linie L1 von einem Anfangspunkt P1 zu einem Endpunkt P2. Während dies für die Aufnahme von Biomasse vorteilhaft ist, kann sich die Schnittebene auch oberhalb bzw. unterhalb des Mittelpunktes erstrecken. Die zweite Linie L2 erstreckt sich auf der Oberfläche des Hohlkörpers 1, hier von der Hohlkugel 1, bogenförmig oder kurvenartig vom Anfangspunkt P1 der Linie L1 zum Endpunkt P2 der Linie L2.

Zudem umfasst der Mischkörper 1 mindestens einen Auftriebskörper 3. Im vorliegenden Ausführungsbeispiel sind zwei Auftriebskörper 3 angeordnet, welche hier auf der Innenseite der Wandung 10 platziert sind. Das Anordnen der Auftriebskörper 3 verhindert ein Absinken der Mischkörper 1, wenn sich diese mit Biomasse B füllen. Vorzugsweise ist einer der Auftriebskörper 3 benachbart zur Öffnung 2 angeordnet. Im vorliegenden Ausführungsbeispiel ist der Auftriebskörper im Bereich der bogenartigen Linie L2 angeordnet. Der andere Auftriebskörper 3 ist diametral gegenüber dem ersten Auftriebskörper angeordnet. Im Ruhezustand liegt der Auftriebskörper derart orientiert in der Biomasse B, dass beide Auftriebskörper in die Biomasse B eintauchen. Aufgrund dieser Orientierung ist auch sichergestellt, dass sich mindestens eine Öffnung 2, mindestens teilweise in der Biomasse B befindet, so dass die Biomasse in den Hohlkörper eintreten kann. Der Mischkörper 1 rotiert aufgrund der Bewegung der Biomasse im wesentlichen um die Achse, welche sich vom Mittelpunkt des einen Hohlkörpers zum Mittelpunkt des anderen Hohlkörpers erstreckt. Aufgrund der Biomasse B im Hohlraum kann aber auch eine Art Taumelbewegung resultieren. Auch sind gemischte, beliebige Bewegungsformen denkbar.

Der Auftriebskörper 3 weist die Gestalt eines Hohlkörpers auf, welcher mit einem Stoff befüllt ist, dessen Dichte kleiner ist als die Dichte der Biomasse. Insbesondere kann der Stoff Luft oder ein anderes Gas sein. Statt eines Hohlkörpers kann auch ein Auftriebskörper aus einem Kunststoff, insbesondere einem geschäumten Kunststoff, wie Polystyrol, vorgesehen sein. Durch das Anordnen eines Auftriebskörpers wird verhindert, dass der Mischkörper 1 in der Biomasse B versinken kann.

Alternativ kann auch der Hohlkörper 1 selbst eine genügend grosse Auftriebskraft bereitstellen. Hierfür ist der Hohlkörper 1 aus einem Material gefertigt, welches auch dann noch eine Auftriebskraft bewirkt, wenn der Hohlkörper 1 mit Biomasse befüllt ist. Beispielsweise ist es denkbar, den gesamten Hohlkörper 1 aus einem geschäumten Kunststoff, wie Polystyrol zu fertigen.

Im vorliegenden Ausführungsbeispiel umfasst der Mischkörper 1 mindestens ein schweifartiges oder kammartiges Mischelement 4. Dieses Mischelement 4 ist auf der Aussenseite der Wandung 10 angeordnet und steht von der Wandung 10 ab. Beim hier dargestellten Mischelement 4 handelt es sich um ein flächiges Element, welches sich über einen Teil der Oberfläche des Mischkörpers 1 erstreckt. Das Mischelement 4 hat nicht nur eine Mischfunktion, bei welcher eine eventuelle Schwimmschicht auf der Oberfläche durchtrennt wird, sondern auch eine Mitnahmefunktion oder Antriebsfunktion. Durch das Vorstehen der Mischelemente 4 über die Oberfläche des Mischkörpers 1 kann die Strömung der Biomasse B an die Mischelemente 4 angreifen, wobei die Rotationsbewegung des Mischkörpers 1 weiter unterstützt wird. Ferner durchtrennen diese Mischelemente 4 eine eventuelle Schwimmschicht. Durch beide Effekte kann der Wirkungsgrad bzw. die Gewinnung von Biogas aus der Biomasse weiterhin erhöht werden.

Im vorliegenden Ausführungsbeispiel erstreckt sich ein erstes Mischelement 4 auf der Oberseite des Hohlkörpers 1 entlang eines ersten Meridians und ein zweites Mischelemente 4 erstreckt sich auf der Unterseite des Hohlkörpers 1 entlang eines zweiten Meridians. Die Äquatorialebene E dient dabei als Trennebene zwischen Unterseite und Oberseite und durchdringt die Kugel durch deren Mittelpunkt. Als ein Meridian wird eine Linie verstanden, welche sich senkrecht von der Äquatorialebene auf der Kugeloberfläche zu einem der Pole erstreckt. Der erste Meridian ist in der Äquatorialebene E winklig zum zweiten Meridian angeordnet. Vorzugsweise beträgt der Winkel vom ersten Meridian zum zweiten Meridian 45° bis 135°. Das Ausführungsbeispiel nach Fig. 3a-3c zeigt eine Anordnung der Meridiane, welche um 90° zueinander versetzt sind. Demnach erstreckt sich ein erstes Mischelement 4 entlang des ersten Meridians über die Oberfläche der Oberseite des Mischkörpers 1 von einem Startpunkt, welcher mit dem Punkt P2 zusammenfällt, zu einem Endpunkt, welcher diametral gegenüber von P2 liegt. Ein weiteres Mischelement 4 erstreckt sich über die Oberfläche der Unterseite des Mischkörpers 1 entlang des zweiten Meridians, von einem Startpunkt, welcher mit dem Punkt P1 zusammenfällt, zu einem Endpunkt, welcher diametral gegenüber von P1 liegt. Alternativ können sich die Mischelemente 4 auch entlang einer beliebigen Kurve auf der Oberfläche des Mischkörpers 1 erstrecken.

In einer alternativen Ausführungsform kann die der Biomasse B zugewandte Oberfläche der Wandung 10 mit einer Oberflächenstruktur versehen sein, welche dem Antrieb des Hohlkörpers 1 und/oder der Durchmischung der Biomasse B dient. Dadurch kann die Biomasse B besonders effektiv durchmischt werden, da bei einer Drehung des Hohlkörpers 1 Teile von der Biomasse B auf der Oberfläche verweilen. Die auf der Oberfläche des Hohlkörpers 1 verweilende Biomasse wird dabei der Luft bzw. dem Biogas ausgesetzt und kann vergären. Beispielsweise ist eine aufgeraute Oberflächenstruktur denkbar. In einer anderen Ausführungsform ist es zudem denkbar, dass eine Vielzahl an stachelartigen Mischelementen von der Oberfläche des Hohlkörpers abstehen. Ferner ist es zudem denkbar, die beschriebenen Oberflächenstrukturen mit dem flächigen Mischelement 4 zu kombinieren.

Ferner ist es zudem denkbar auch die dem Hohlraum 11 zugewandte Oberfläche der Wandung 10 mit einer entsprechenden Oberflächenstruktur zu versehen. Dadurch kann die Biomasse, welcher sich im Hohlkörper befindet, ebenfalls gut durchmischt werden.

Die Hohlkugel 1 weist typischerweise einen Durchmesser im Bereich von 20 cm bis 180 cm, insbesondere zwischen 30 cm und 150 cm auf. Falls der Hohlkörper 1 eine andere Gestalt aufweist, im Querschnitt beispielsweise oval, elliptisch rechteckig oder quadratisch ist, beziehen sich diese Abmessungen auf die maximale Ausdehnung des Hohlkörpers.

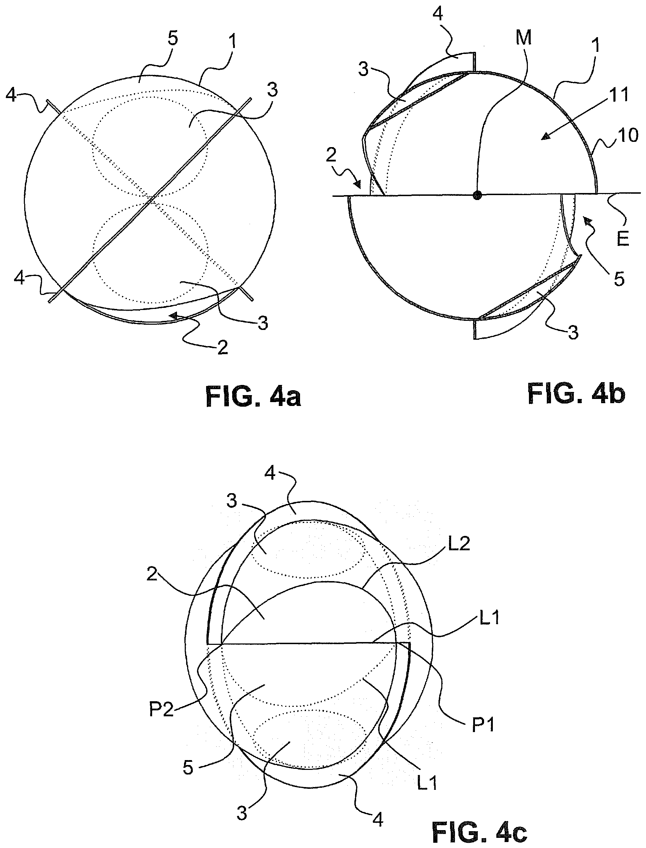

Die Figuren 4a bis 4c zeigen eine zweite Ausführungsform des erfindungsgemässen Mischkörpers 1. Gleiche Teile sind mit gleichen Bezugszeichen bezeichnet. Ferner können Teilmerkmale aller beschriebenen Ausführungsbeispiele beliebig untereinander zu weiteren Ausführungsbeispielen kombiniert werden.

Die zweite Ausführungsform des Mischkörpers 1, welcher hier ebenfalls als Hohlkugel ausgebildet ist, umfasst eine weitere Öffnung 5. Vorzugsweise ist die weitere Öffnung 5 diametral zur ersten Öffnung 1 angeordnet. Das heisst mit anderen Worten, dass sich die erste Öffnung 2 von der Ebene E nach oben erstreckt und dass sich die zweite Öffnung 5 von der Ebene E nach unten erstreckt. Ferner ist die weitere Öffnung 5 vorzugsweise mit identischer Form und Abmessung zur ersten Öffnung 2 ausgebildet. Durch das Anordnen einer weiteren Öffnung 5 kann über einen gleichen Zeitraum ein grössere Menge Biomasse B in den Hohlraum einströmen und diesen verlassen. Dadurch kann die Effektivität des erfindungsgemässen Mischkörpers weiter gesteigert werden.

In weiteren Ausführungsformen können zudem weitere Öffnungen angeordnet werden. Alternativ ist es auch möglich, einen Mischkörper bzw. einen Hohlkörper 1 bereitzustellen, welcher keine Öffnungen aufweist. Man spricht dabei von einem geschlossenen Mischkörper. Ein solcher Mischkörper kann hohl ausgebildet sein oder er kann mit einem Material befüllt sein, welches eine geringere Dichte als die Biomasse aufweist, so dass der Mischkörper auf der Biomasse aufschwimmt. Alternativ kann ein solcher Mischkörper zudem ein Mischelement 4, wie oben beschrieben, umfassen.

Vorzugsweise ist der Mischkörper bzw. der Hohlkörper 1 aus einem Kunststoff gefertigt, welcher gegenüber der Biomasse und der aus der Biomasse resultierenden Biogase resistent ist. Beispiel für einen Kunststoff sind: Polypropylen, Polyethylen oder HDPE, Acrylnitril-Butadien-Styrol, etc.

Der erfindungsgemässe Mischkörper 1 kann beispielsweise mittels eines Spritzgussverfahrens hergestellt werden. Hierfür kann der Mischkörper 1 auch aus zwei Teilen oder Halbschalen ausgebildet sein, nämlich aus einem oberen Teil, welcher sich oberhalb der Ebene E erstreckt, und einem unteren Teil, welcher sich unterhalb der Ebene E erstreckt. Die beiden Teile können dann miteinander verbunden, insbesondere verklebt oder verschweisst, werden. Alternativ kann auch der gesamte Mischkörper 1 einstückig hergestellt werden.

In einer alternativen Ausführungsform kann der Mischkörper 1 auch aus nicht-rostendem Stahl oder Aluminium, dessen Oberfläche optional eloxiert ist, oder anderen metallischen Werkstoffen gefertigt sein. Auch hier kann der Mischkörper 1 beispielsweise aus zwei Halbschalen, wie oben beschrieben, gefertigt sein, welche dann miteinander verbunden werden.

### Bezugszeichenliste

- 1: Mischkörper, Hohlkörper
- 10: Wandung
- 11: Hohlraum
- 2: erste Öffnung
- 3: Auftriebskörper
- 4: Mischelement
- 5: zweite Öffnung

- L1: erste Linie
- L2: zweite Linie
- P1: Anfangspunkt
- P2: Endpunkt
- W: Seitenwand
- D: Deckel
- B: Biomasse
- G: Gasgemisch
- O: Oberfläche der Biomasse
- M: Mittelpunkt
- E: Ebene

## Patentansprüche

1. Verfahren zur Herstellung von Biogas, bei welchem Biomasse in einen Biogasreaktor (R) eingefüllt wird, **dadurch gekennzeichnet, dass** eine Vielzahl von sich frei bewegenden Mischkörpern (1) auf der Biomasse (B) angeordnet werden, die auf der Oberfläche (O) der Biomasse (B) schwimmen und dabei teilweise in die Biomasse (B) eintauchen.

2. Verfahren zur Herstellung von Biogas nach Anspruch 1, **dadurch gekennzeichnet, dass** die Mischkörper (1) zwischen 20% bis 85%, insbesondere 30% bis 70% der Oberfläche (O) der Biomasse (B) überdecken.

3. Verfahren nach einem der vorhergehenden Ansprüche, **dadurch gekennzeichnet, dass** die Mischkörper (1) als Hohlkörper ausgebildet sind und/oder eine Oberflächenstruktur (4) aufweisen und/oder im wesentlichen kugelförmig sind.

4. Mischkörper (1) zur Verwendung in einem Verfahren nach einem der vorhergehenden Ansprüche, **dadurch gekennzeichnet, dass** der Mischkörper (1) als Hohlkörper mit einer kugelartigen Grundform und mit mindestens einer Öffnung (2, 5) ausgebildet ist, wobei die mindestens eine Öffnung (2, 5) ein Eintreten bzw. ein Austreten von Biomasse (B) in den Hohlkörper erlaubt, wobei sich die mindestens eine Öffnung (2, 5) über einen Oberflächenabschnitt erstreckt, welcher durch eine erste Linie (L1) und eine zweite Linie (L2) begrenzt wird, wobei die erste Linie (L1) eine Schnittlinie auf der Oberfläche des Hohlkörpers mit einer Ebene (E) ist, wobei sich die erste Linie von einem Anfangspunkt (P1) zu einem Endpunkt (P2) erstreckt, und wobei die zweite Linie (L2) eine bogenartige Linie ist, welche sich auf der Oberfläche des Hohlkörpers vom Anfangspunkt (P1) zum Endpunkt (P2) der ersten Linie (L1) erstreckt.

5. Mischkörper (1) nach Anspruch 4, **dadurch gekennzeichnet, dass** der Hohlkörper mindestens eine weitere Öffnung (5) aufweist.

6. Mischkörper (1) nach einem der Ansprüche 4 bis 7, **dadurch gekennzeichnet, dass** die weitere Öffnung (5) im wesentlichen diametral gegenüber der ersten Öffnung (2) angeordnet ist.

7. Mischkörper (1) nach einem der Ansprüche 4 bis 6, **dadurch gekennzeichnet, dass** der Mischkörper (1) mindestens ein Mischelement (4) aufweist, welches auf der Aussenseite der Mischkörper angeordnet ist.

8. Mischkörper (1) nach Anspruch 7, **dadurch gekennzeichnet, dass** sich ein erstes längliches Mischelement (4) auf der Oberseite des Hohlkörpers (1) entlang eines ersten Meridians erstreckt, und dass ein zweites längliches Mischelement auf der Unterseite des Hohlkörpers (1) entlang eines zweiten Meridians erstreckt, wobei das erste Mischelemente (4) und das zweite Mischelement (4) in der Äquatorialebene (E), winklig zueinander versetzt angeordnet sind.

9. Mischkörper (1) nach einem der Ansprüche 7 bis 8, **dadurch gekennzeichnet, dass** der Winkel zwischen den beiden Mischelementen (4) in der Äquatorialebene (E) zwischen 45° und 135° ist.

10. Mischkörper (1) nach einem der Ansprüche 4 bis 9, **dadurch gekennzeichnet, dass** der Mischkörper (1) mindestens einen Auftriebskörper (3) umfasst, wobei der mindestens eine Auftriebskörper (3) vorzugsweise im Inneren des Hohlkörpers (1) angeordnet ist.

11. Mischkörper (1) nach Anspruch 10, **dadurch gekennzeichnet, dass** der mindestens eine Auftriebskörper (3) einen Hohlraum aufweist, welcher mit einem Medium befüllt ist, das eine geringere Dichte als die Biomasse aufweist.

12. Mischkörper (1) nach einem der Ansprüche 10 bis 11, **dadurch gekennzeichnet, dass** das Medium, mit welchem der mindestens eine Auftriebskörper (3) befüllt ist, ein Gas, insbesondere Luft, oder ein geschäumter Kunststoff ist.

13. Mischkörper (1) nach einem der Ansprüche 10 bis 12, **dadurch gekennzeichnet, dass** der mindestens eine Auftriebskörper (3) einstückig mit der Wandung (10) des Hohlkörpers (1) ausgebildet ist.

## Claims

1. A method for producing biogas, in which biomass is introduced into a biogas reactor (R), **characterized in that** a multiplicity of freely moving mixing bodies (1) are arranged on the biomass (B), which float on the surface (O) of the biomass (B) and at the same time dip partially into the biomass (B).

2. The method for producing biogas as claimed in claim 1, **characterized in that** the mixing bodies (1) cover between 20% and 85%, in particular between 30% and 70%, of the surface (O) of the biomass (B).

3. The method as claimed in one of the preceding claims, **characterized in that** the mixing bodies (1) are designed as hollow bodies and/or have a surface structure (4) and/or are essentially spherical.

4. A mixing body (1) for use in a method as claimed in one of the preceding claims, **characterized in that** the mixing body (1) is designed as a hollow body with a spherical base form and with at least one orifice (2, 5), the at least one orifice (2, 5) allowing entry and exit of biomass (B) into the hollow body, wherein the at least one orifice (2, 5) extends over a surface portion which is delimited by a first line (L1) and a second line (L2), the first line (L1) being a line of intersection on the surface of the hollow body with a plane (E), the first line extending from a starting point (P1) to an end point (P2), and the second line (L2) being an arcuate line which extends on the surface of the hollow body from the starting point (P1) to the end point (P2) of the first line (L1).

5. The mixing body (1) as claimed in claim 4, **characterized in that** the hollow body has at least one further orifice (5).

6. The mixing body (1) as claimed in either one of claims 4 and 5. **characterized in that** the further orifice (5) is arranged essentially diametrically opposite the first orifice (2).

7. The mixing body (1) as claimed in one of claims 4 to 6, **characterized in that** the mixing body (1) has at least one mixing element (4) which is arranged on the outside of the mixing bodies.

8. The mixing body (1) as claimed in claim 7, **characterized in that** a first elongate mixing element (4) extends on the top side of the hollow body (1) along a first meridian, and **in that** a second elongate mixing element extends on the underside of the hollow body (1) along a second meridian, the first mixing element (4) and the second mixing element (4) being arranged, offset at an angle with respect to one another, in the equatorial plane (E).

9. The mixing body (1) as claimed in either one of claims 7 and 8, **characterized in that** the angle between the two mixing elements (4) in the equatorial plane (E) is between 45° and 135°.

10. The mixing body (1) as claimed in one of claims 4 to 9, **characterized in that** the mixing body (1) comprises at least one buoyancy body (3), wherein the at least one buoyancy body (3) is preferably arranged inside the hollow body (1).

11. The mixing body (1) as claimed in claim 10, **characterized in that** the at least one buoyancy body (3) has a cavity filled with a medium which has a lower density than the biomass.

12. The mixing body (1) as claimed in one of claims 10 to 11, **characterized in that** the medium with which the at least one buoyancy body (3) is filled is a gas, in particular air, or a foamed plastic.

13. The mixing body (1) as claimed in one of claims 10 to 12, **characterized in that** the at least one buoyancy body (3) is formed in one piece with the wall (10) of the hollow body (1).

## Revendications

1. Un procédé pour fabriquer du biogaz, dans lequel de la biomasse est versée dans un réacteur à biogaz (R), **caractérisé en ce qu'**une pluralité de corps de mélange (1) librement mobiles sont agencés sur la biomasse (B), qui flottent à la surface (O) de la biomasse (B) et sont ainsi partiellement immergés dans la biomasse (B).

2. Un procédé pour fabriquer du biogaz selon la revendication 1, **caractérisé en ce que** les corps de mélange (1) recouvrent de 20% à 85%, préférablement de 30% à 70% de la surface (O) de la biomasse (B).

3. Un procédé pour fabriquer du biogaz selon une des revendications précédentes, **caractérisé en ce que** les corps de mélange (1) sont formées en tant que corps creux et/ou présentent une structure de surface (4) et/ou sont sensiblement sphériques.

4. Un corps de mélange (1) pour l'utilisation dans un procédé selon une des revendications précédentes, **caractérisé en ce que** le corps de mélange (1) est formé en tant que corps creux présentant une forme de base sensiblement sphérique et avec au moins une ouverture (2, 5), où l'au moins une ouverture (2, 5) permet l'entrée respectivement la sortie de biomasse (B) dans le corps creux, où l'au moins une ouverture (2, 5) s'étend sur un segment de surface qui est délimité par une première ligne (L1) et une deuxième ligne (L2), où la première ligne (L1) est une ligne d'intersection sur la surface du corps creux avec un plan (E), où la première ligne s'étend depuis un premier point de départ (P1) vers un second point terminal (P2), où la deuxième ligne (L2) est une ligne arquée, laquelle s'étend sur la surface du corps creux depuis le point de départ (P1) vers le point terminal (P2) de la première ligne (L1).

5. Le corps de mélange (1) selon la revendication 4, **caractérisé en ce que** le corps creux présente au moins une ouverture (5) supplémentaire.

6. Le corps de mélange (1) selon une des revendications 4 à 5, **caractérisé en ce que** l'ouverture (5) supplémentaire est disposée sensiblement diamétralement opposée à la première ouverture (2).

7. Le corps de mélange (1) selon une des revendications 4 à 6, **caractérisé en ce que** le corps de mélange (1) présente au moins un élément de mélange (4), lequel est disposé à l'extérieur du corps de mélange.

8. Le corps de mélange (1) selon la revendication 7, **caractérisé en ce qu'**un premier élément de mélange (4) allongé s'étend le long d'un premier méridien sur le côté supérieur du corps creux (1), et qu'un deuxième élément de mélange allongé s'étend le long d'un deuxième méridien sur le côté inférieur du corps creux (1), où le premier élément de mélange (4) et le deuxième élément de mélange (4) sont disposés de manière décalée de façon angulaire l'un par rapport à l'autre dans le plan équatorial (E).

9. Le corps de mélange (1) selon une de revendications 7 à 8, **caractérisé en ce que** l'angle entre les deux éléments de mélange (4) dans le plan équatorial (E) se situe entre 45° et 135°.

10. Le corps de mélange (1) selon une de revendications 4 à 9, **caractérisé en ce que** le corps de mélange (1) comporte au moins un corps de flottaison (3), ou l'au moins un corps de flottaison (3) est disposé préférablement à l'intérieur du corps creux (1).

11. Le corps de mélange (1) selon la revendication 10, **caractérisé en ce que** l'au moins un corps de flottaison (3) comporte au moins une cavité, laquelle est rempli d'un milieu ayant une densité inférieure à celle de la biomasse.

12. Le corps de mélange (1) selon une des revendications 10 à 11, **caractérisé en ce que** le milieu, avec lequel l'au moins un corps de flottaison (3) est rempli, est un gaz, en particulier de l'air ou une résine alvéolaire.

13. Le corps de mélange (1) selon une des revendications 10 à 12, **caractérisé en ce que** l'au moins un corps de flottaison (3) est formé en une pièce sur la paroi (10) du corps creux (1).
